# EUROPEAN PATENT APPLICATION

(11) **EP 1 364 962 A1**
(43) Date of publication of application: **26.11.2003**
(21) Application number: 02077061.6
(22) Date of filing: 24.05.2002
(51) Int. Cl.: C07K 14/47, A61K 38/17, A61K 45/00, A61K 48/00, G01N 33/50, A61P 5/14, A61K 38/00, A61K 39/395

(54) **Use of monocarboxylate transporter proteins for thyroid hormone transport**

(71) Applicant: Stichting tot bevordering van de wetenschap der Endocrinologie, 3015 GD Rotterdam (NL)
(72) Inventor: Friesema, Edith, Catharina, Hendrika, 3176 TE Poortugaal (NL); Krenning, Eric, Paul, 3062 JC Rotterdam (NL); Visser, Theofilus, Johannes, 3172 VW Poortugaal (NL)
(74) Representative: Prins, Adrianus Willem

(57) **Abstract**

The invention provides a use of a monocarboxylate transporter protein or a functional part, derivative and/or analogue thereof for altering transport of a thyroid hormone or a functional part, derivative and/or analogue thereof across a membrane. Said monocarboxylate transporter protein preferably comprises MCT-8. An isolated molecule capable of specifically binding at least part of an MCT protein, or at least part of a ligand of an MCT protein, is also herewith provided. Regulation of the bioavailability of thyroid hormone in a tissue enables interfering with (metabolic) diseases. Hence, the invention also provides pharmaceutical compositions comprising a compound capable of binding said MCT protein, or capable of influencing the binding or transporting of a ligand of said MCT protein. Methods for treatment of a disease such as a disorder of thyroid metabolism, non-thyroidal illness, obesity or cardiovascular illness are also provided, as well as bioassays for identifying or detecting a candidate drug capable of binding to or influencing at least part of said MCT protein.

## Description

The invention relates to the field of endocrinology, more specifically to the field of regulating thyroid hormone bioavailability and activity.

Thyroid hormone is essential for the development and homeostasis of different organs, particularly for the regulation of energy metabolism. The follicular cells of the thyroid gland produce predominantly the prohormone thyroxine (3,3',5,5'-tetraiodothyronine, T4) which has little or no biological activity. T4 is activated by enzymatic outer ring deiodination to 3,3',5-triiodothyronine (T3), which is the most if not only bioactive form of thyroid hormone (1-4). Both T4 and T3 are inactivated by enzymatic inner ring deiodination to the metabolites 3,3',5'-triiodothyronine (reverse T3, rT3) and 3,3'-diiodothyronine (3,3'-T2), respectively (1-4).
The three deiodinases involved in these processes show different catalytic profiles, tissue distributions and regulatory functions. They have recently been identified as homologous transmembrane selenoproteins with their active site exposed to the cytoplasm (1-4).
Additional pathways of thyroid hormone metabolism include sulfation and glucuronidation of the phenolic hydroxyl group by transferases located in the cytoplasm and endoplasmic reticulum of different tissues (2,4).
Thyroid hormone is essential for the development of different tissues, for example the brain, and for the regulation of energy metabolism of a wide range if not all tissues throughout life (5) and regulating its bioavailability or activity in various tissues would enable regulating, influencing or interfering with (metabolic) disease in those tissues.
Considering the central role that is played by thyroid hormone in the basal metabolism of a wide variety of cells in a wide range of tissues, use of drugs comprising thyroid hormone agonists and/or antagonists will have beneficial effects in many diseases.
Access of plasma thyroid hormone to intracellular receptors and enzymes requires transport across the cell membrane. On the basis of the lipophilic nature of iodothyronines, it was assumed for a long time that they cross the cell membrane by simple diffusion. However, this ignored the highly polar nature of the alanine side chain which is a formidable obstacle for membrane passage of iodothyronines. During the last two decades, although none were found, overwhelming evidence has accumulated indicating the involvement of plasma membrane transporters in tissue uptake of thyroid hormone (4,6).
In EP 0982399 we reported that such plasma membrane thyroid transporters indeed exist. In that patent application plasma membrane thyroid transporters are provided, such as polypeptides related to an organic anion transporting peptide. These peptides comprise sodium-dependent taurocholate co-transporting polypeptide or sodium-independent organic anion-transporting peptide. An example of such a polypeptide is rat Ntcp, which is a 362-aminoacid protein containing 7 putative transmembrane domains and 2 glycosylation sites with an apparent molecular mass of 51 kDa (7,9,10). It and its orthologues are only expressed in differentiated mammalian hepatocytes, where it is localized selectively to the basolateral cell membrane (9, 10). It is the major transporter of conjugated bile acids in liver but it also mediates uptake of unconjugated bile acids (9, 10 11). The reported Km values of taurocholate for rat Ntcp vary between 15 and 51 µM (10). Ntcp may also mediate transport of a number of non-bile acid amphipathic compounds, including estrogen conjugates such as estrone 3-sulfate (11).

Another example is rat oatp 1 which is a 670-aminoacid protein with 12 transmembrane domains and 2 glycosylation sites with an apparent molecular mass of 80 kDa (8-10). Oatp1 is not only expressed in liver but also in kidney and brain. Like Ntcp, oatp1 is localized to the basolateral liver cell membrane. Oatp1 is a multispecific transporter mediating the uptake of a wide variety of amphipathic ligands (9, 10, 12-15), including conjugated and unconjugated bile acids, conjugated steroids (*e.g*. estrone sulfate, estradiol 17β-glucuronide and DHEA sulfate) and other organic anions (*e.g*. the prototypic bromosulfophthalein), but also neutral steroids (*e.g*. aldosterone and cortisol), cardiac glycosides (*e.g*. ouabain) and even organic cations (*e.g*. ajmalinium). Apparent Km values of taurocholate and bromosulfophthalein for rat oatp1 amount to 50 and 1.5 µM, respectively (10). In contrast to Ntcp, transport through oatp1 is not coupled to Na⁺. Various members of the oatp transporter family in humans and rats have now been shown to be capable of transporting thyroid hormone (16)

It is an object of the present invention to provide alternative plasma membrane thyroid transporters.

Surprisingly it has been found by the present inventors that a monocarboxylate transporter protein (MCT) is capable of transporting a thyroid hormone across a plasma membrane. This class of proteins is reported to transport monocarboxylates such as lactate and pyruvate (Reviewed in (17)). Until now, no correlation with thyroid hormone transport was known.

The invention provides a use of a monocarboxylate transporter (MCT) protein or a functional part, derivative and/or analogue thereof for altering transport of a thyroid hormone or a functional part, derivative and/or analogue thereof across a membrane. Preferably, said transport is enhanced. When the term "MCT protein" is mentioned in the description, it can also refer to a functional part, derivative and/or analogue of an MCT protein.
Transport of a thyroid hormone across a membrane can for instance be enhanced by providing MCT protein to said membrane. Uptake of said MCT protein in said membrane can increase the concentration of said MCT protein. A higher concentration of MCT can result in increased transport of thyroid hormone across said membrane.

Transport of a thyroid hormone can also be altered by a molecule capable of specifically binding a monocarboxylate transporter protein or a functional part, derivative and/or analogue thereof, or a ligand of said monocarboxylate transporter protein. Such isolated molecule is therefore also herewith provided. In one embodiment, said molecule comprises a (poly)peptide. A molecule with specific binding properties can be generated and/or identified by methods known in the art. For instance, one can use Pepscan techniques and/or replacement mapping techniques as well as for instance phage-display techniques and screening of combinatorial libraries, allowing identification of active sites in a polypeptide sequence.
A molecule capable of specifically binding at least part of an MCT protein, or capable of specifically binding at least part of a ligand of an MCT protein, can for instance act as an antagonist, decreasing transport of thyroid hormone across a membrane by said MCT protein. Alternatively, said molecule can act as an agonist, enhancing thyroid hormone transport. The invention thus provides a use of a molecule of the invention for altering transport of a thyroid hormone or a functional part, derivative and/or analogue thereof across a membrane. Preferably a use of the invention is provided wherein said membrane comprises a plasma membrane.

In a preferred embodiment said monocarboxylate transporter protein comprises monocarboxylate transporter protein-8. As is illustrated by the examples, MCT-8 shows very good thyroid hormone transporter activity. Transport of thyroid hormone across a plasma membrane appears even to be carried out more efficiently by MCT-8 as compared to currently known Ntcp and oatp transporters. However, other members of the MCT family such as MCT-1, MCT-2, MCT-3, MCT-4, MCT-5, MCT-6, MCT-7 and MCT-9 are also within the scope of the present invention.

A functional part of a protein is defined as a part which has the same kind of properties in kind, not necessarily in amount. A functional derivative of a protein is defined as a protein which has been altered such that the properties of said molecule are essentially the same in kind, not necessarily in amount. A derivative can be provided in many ways, for instance through conservative amino acid substitution.
A person skilled in the art is well able to generate analogous compounds of a protein. This can for instance be done through screening of a peptide library. Such an analogue has essentially the same properties of said protein in kind, not necessarily in amount.

In one aspect the invention provides a compound capable of influencing the binding or transporting of a ligand of, or capable of binding to, a plasma membrane polypeptide capable of transporting a thyroid hormone, wherein said polypeptide comprises a monocarboxylate transporter protein or a functional part, derivative and/or analogue thereof, for use as a medicament. Preferably, said polypeptide is capable of transporting 3,3',5-triiodothyronine. More preferably, said monocarboxylate transporter protein comprises monocarboxylate transporter protein-8.

A compound as provided by the invention is suitable for use in methods for treating a wide range of disorders, such as disorders of thyroid hormone metabolism, for example related to brain disorders seen with psychiatric disease, to restore or help develop tissue metabolism or development in premature children, to help restore thyroid hormone function in patients wherein the thyroid has been removed or is dysfunctioning, for example due to a malignancy, to help alleviate non-thyroidal illness, to treat obesity or cardiovascular illness, to name a few. In fact, all disorders or diseases wherein the (basal) metabolism of a cell or tissue is affected can be treated with a compound of the invention, which can act as an agonist or as an antagonist. An agonist mainly acts in up-regulating metabolism and an antagonist mainly acts in down-regulating metabolism. In a preferred embodiment, such a compound of the invention comprises a peptide, preferably a synthetic peptide, or an antibody or other binding molecule or thyroid hormone analogue capable of binding to or influencing or interfering with the binding or transporting of a ligand (for example T3) of MCT. A compound of the invention is preferably capable of binding at least part of MCT, and/or at least part of a ligand of MCT. Optionally a compound as provided by the invention is provided with a carrier known in the art for production of a medicament. Said carrier may be a diluent.
Compounds comprising tissue specific thyroid hormone agonists and/or antagonists as provided by the invention can for example be used in treating obesity, heart failure or (tissue specific) hypo- or hyper-thyroidism. Another example is when tissue specific malignancies such as tumours require up- or down-regulation. Such malignancies can be treated by determining which thyroid hormone transporter is used by the cells in the tissue or malignancy, (for example by biopsy and histochemistry using immunological detection or detection of mRNA expression) and then treating the patient with an agonist/antagonist as provided by the invention which specifically acts through the then determined transporter. Furthermore, compounds comprising (tissue specific) thyroid hormone agonists/antagonists as provided by the invention can be used for the production of a medicament for treating a disorder of thyroid metabolism, non-thyroidal illness, obesity or cardiovascular illness.
Thus, the invention provides a use of a compound of the invention for the production of a medicament for the treatment of a thyroid hormone related disorder, non-thyroidal illness, obesity or cardiovascular illness.

A pharmaceutical composition comprising a compound of the invention and a suitable carrier is also herewith provided. Such pharmaceutical compound is particularly suitable for treating a disorder such as a disorder of thyroid metabolism, non-thyroidal illness, obesity or cardiovascular illness. In one embodiment the invention therefore provides a method for treating a disorder such as a disorder of thyroid metabolism, non-thyroidal illness, obesity or cardiovascular illness, comprising administering a compound or a pharmaceutical composition of the invention to an individual suffering from said disorder. Said compound or pharmaceutical composition can for instance be administered to an individual orally, by aerosol or as a suppository. Alternatively, said compound can be administered with aid of gene therapy, involving administration of a nucleic acid encoding at least part of said compound, and expression and translation of said nucleic acid, preferably in a host cell. Said nucleic acid for instance comprises DNA or RNA. Methods for the preparation and administration of a compound or pharmaceutical composition are known in the art, as well as suitable carriers for pharmaceutical compositions. Likewise, methods for gene therapy are known by the person skilled in the art. In the art, many vectors and protocols are provided allowing the person skilled in the art to perform an optimal therapy for each application.
A use of at least a functional part of a monocarboxylate transporter protein, or of a nucleic acid encoding at least a functional part of a monocarboxylate transporter protein, in a method for treating a disorder such as a thyroid hormone related disorder such as a disorder of thyroid hormone metabolism, non-thyroidal illness, obesity or cardiovascular illness is also herewith provided. In one embodiment, said use comprises gene therapy.

In yet another aspect the invention provides a method for altering transport of a thyroid hormone or a functional part, derivative and/or analogue thereof across a membrane comprising providing said membrane with a monocarboxylate transporter protein or a functional part, derivative and/or analogue thereof, and/or with a molecule capable of specifically binding a monocarboxylate transporter protein or a functional part, derivative and/or analogue thereof, or a ligand of said monocarboxylate transporter protein. As has been outlined before, an MCT protein and a molecule capable of specifically binding at least part of an MCT protein or at least part of a ligand of an MCT protein are particularly suitable for altering thyroid hormone transport across a membrane, preferably across a plasma membrane. In a preferred embodiment said MCT protein comprises MCT-8.

An MCT protein or a nucleic acid encoding at least a functional part of an MCT protein, preferably an MCT-8 protein, can be used in a method for detecting a pharmaceutical compound. The identification of MCT protein involved in the uptake of thyroid hormone is not only important in the study of the regulation of these processes in health and disease, but it also provides for the detection and/or development of thyroid hormone agonists and antagonists which are beneficial in the treatment of conditions such as obesity and cardiovascular diseases. Ligands or blockers of MCT proteins are important candidate drugs for the development of pharmaceutical compositions acting as a (tissue specific) agonist or antagonist of thyroid hormone activity, which are herewith provided. Methods to identify and/or generate a ligand or blocker of a protein, such as an MCT protein, are known in the art. One can for instance use an assay allowing thyroid hormone and a candidate drug compound to compete for transport across a membrane by an MCT protein. One can also provide a host cell with a nucleic acid sequence encoding at least part of said protein and allow for expression of said nucleic acid sequence. Transport of thyroid hormone in the presence of a candidate drug compound across the membrane of said host cell can subsequently be determined. The invention therefore provides a use of at least a functional part of a monocarboxylate transporter protein, or a nucleic acid encoding at least a functional part of a monocarboxylate transporter protein, in a method for detecting and/or generating a pharmaceutical compound. A preferred embodiment provides a use of the invention wherein said monocarboxylate transporter protein comprises monocarboxylate transporter protein-8.

In one embodiment, such ligands or blockers comprise a peptide derived from a nucleic acid or fragment thereof encoding an MCT protein. To derive peptides which act as ligand (agonist) or blocker (antagonist) is a skill known in the art, for example, one can use Pepscan techniques or replacement mapping techniques as well as for example phage-display techniques and screening of combinatorial libraries, allowing identification of active sites in a polypeptide sequence. In particular, the invention provides a peptide at least comprising an active site capable of binding to or influencing or interfering with the binding and/or transporting of a ligand (preferably of a thyroid hormone nature or functionally equivalent thereto) of a thyroid hormone binding site or part thereof, of an MCT protein, preferably MCT-8.
Furthermore, the invention provides a (synthetic) antibody or other binding molecule specifically directed against an MCT protein, preferably MCT-8, or at least binding to or interfering with the binding of a ligand of a thyroid hormone binding site or parts thereof, of said MCT protein. Generating antibodies or other binding molecules is a skill available in the art, and can be done with classical immunological techniques as well as for example with phage-display techniques.

The invention also provides a bioassay or method to identify said candidate drug agonists or antagonists, for example for use in a pharmaceutical composition for treating obesity, heart failure or (tissue specific) hypo- or hyper-thyroidism. Candidate drugs, often first selected or generated via combinatorial chemistry or comprising a peptide as provided by the invention, can now be tested and identified using a method provided by the invention. Such a candidate drug or compound can for example be tested on and selected for its effect on T3 uptake by an MCT protein. For use in brain cells, where T3 is autonomously produced, a candidate drug or compound can for example be tested on and selected for its effect on T4 uptake as a precursor for T3. As for example can be seen in several of the figures in the description, the invention provides methods and means to measure thyroid hormone cellular uptake by an MCT protein, and regulation thereof. The invention thus provides in one aspect a bioassay to identify or detect a candidate drug capable of binding to or influencing a plasma membrane polypeptide capable of transporting a thyroid hormone, wherein said polypeptide comprises a monocarboxylate transporter protein or a functional part, derivative and/or analogue thereof.
In a preferred embodiment, the invention provides a bioassay of the invention using at least a functional part of a monocarboxylate transporter protein, a nucleic acid encoding at least a functional part of a monocarboxylate transporter protein, a molecule capable of specifically binding at least part of said monocarboxylate transporter protein or a ligand thereof, a cell comprising said nucleic acid and/or a cell comprising at least part of said monocarboxylate transporter protein.
In a most preferred embodiment a bioassay of the invention is provided wherein said monocarboxylate transporter protein comprises monocarboxylate transporter protein-8.

The invention also provides an isolated or recombinant monocarboxylate transporter-8 protein or a functional part, derivative and/or analogue thereof. Such functional part for instance comprises a thyroid hormone binding site or at least a part of such a site, which is often comprising one or more peptides within the large polypeptide. Expressing recombinant protein and isolating and purifying a protein or fragment thereof is a skill available in the art. For instance, a vector can be provided with a nucleic acid sequence encoding a monocarboxylate transporter-8 protein or a functional part, derivative and/or analogue thereof. Said vector can be administered to a host cell, for instance by a gene delivery vehicle. If said cell is capable of expressing said MCT protein, said MCT protein can be produced and isolated by methods known in the art.
A vector comprising a nucleic acid encoding a monocarboxylate transporter protein, preferably MCT-8, or a functional part, derivative and/or analogue thereof is also suitable for gene therapy. The same is true for a vector comprising a nucleic acid sequence encoding a molecule capable of specifically binding a monocarboxylate transporter protein, preferably MCT-8, or a functional part, derivative and/or analogue thereof, or for a vector capable of specifically binding a ligand of said monocarboxylate transporter protein. Such vectors are therefore also herewith provided.
The invention also provides a gene delivery vehicle comprising a vector of the invention. Methods for generating a gene delivery vehicle comprising a certain nucleic acid of interest are known in the art. A gene delivery vehicle of the invention is very suitable for treatment of a disorder such as a disorder of thyroid metabolism, non-thyroidal illness, obesity or cardiovascular illness with gene therapy.

The invention is further explained in the following examples. The examples do not limit the scope of the invention; they merely serve to exemplify the invention

### Examples

### Example 1. Transport of iodothyronines by MCT8 in oocytes

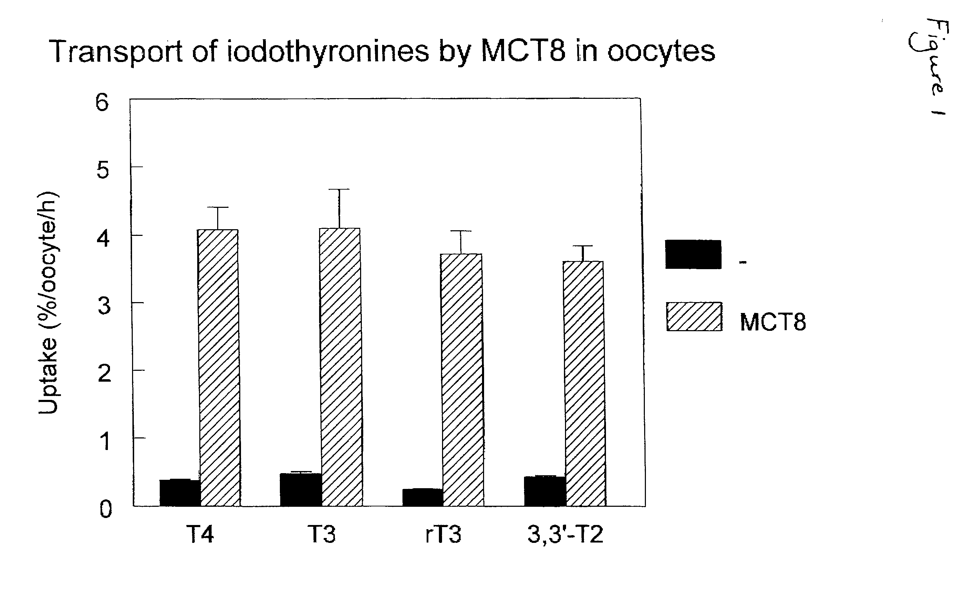

Xenopus oocytes were isolated and injected with 4.6 ng rat MCT8 cRNA. After 3 days, groups of 10 injected or uninjected oocytes were incubated for 60 min at 25 C with 10 nM radioactive iodothyronines in 0.1 ml medium. Uptake of iodothyronines was determined as previously described (18). The results are shown in figure 1.

### Example 2 Transport of amino acids by MCT8 in oocytes

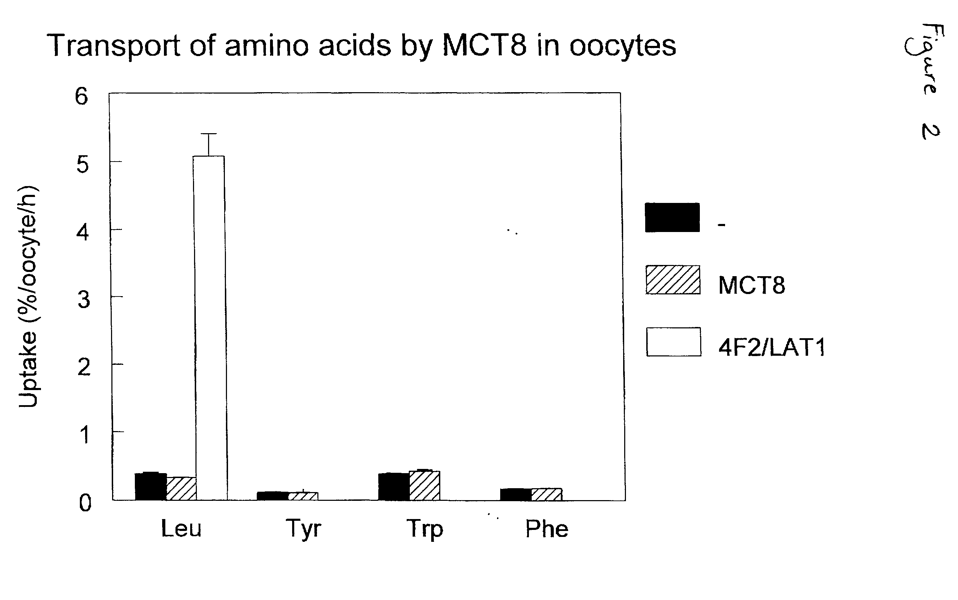

Xenopus oocytes were isolated and injected with cRNA coding for rat MCT8 or with cRNAs coding for the heavy chain (4F2) and the light chain (LAT1) of the heterodimeric human L-type amino acid transporter. After 3 days, groups of 10 injected or uninjected oocytes were incubated for 60 min at 25 C with 10 µM radioactive Leu, Tyr, Trp or Phe in 0.1 ml medium. Amino acid uptake was determined as previously described (18). The results are shown in figure 2.

### Example 3. Saturation of T4 transport by MCT8 in oocytes

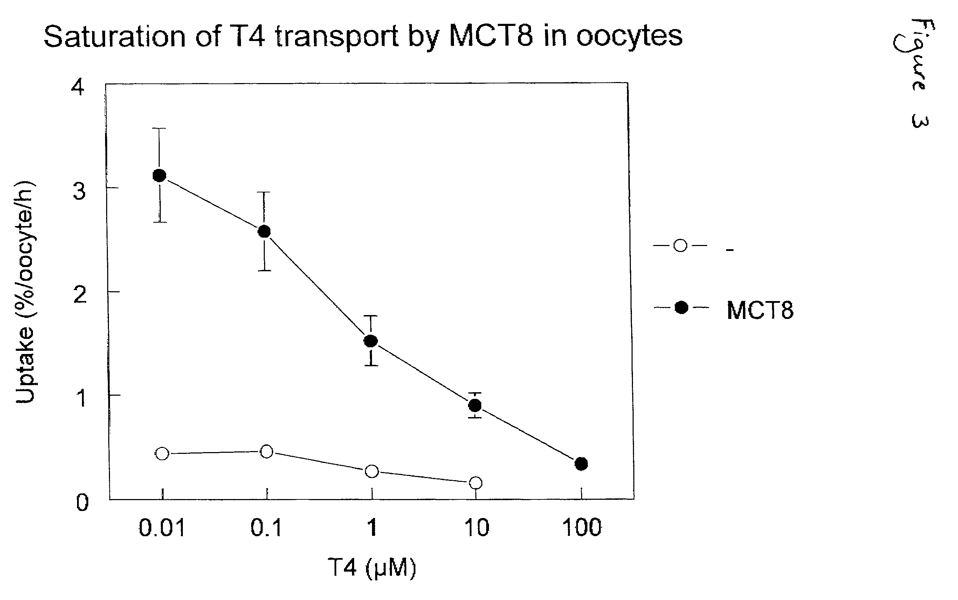

Xenopus oocytes were isolated and injected with rat MCT8 cRNA. After 3 days, groups of 10 injected or uninjected oocytes were incubated for 60 min at 25 C with radioactive T4 and increasing concentrations of nonradioactive T4 in 0.1 ml medium. T4 uptake was determined as previously described (18). The results are shown in figure 3.

### Example 4. Time course of T3 uptake by MCT8 in oocytes

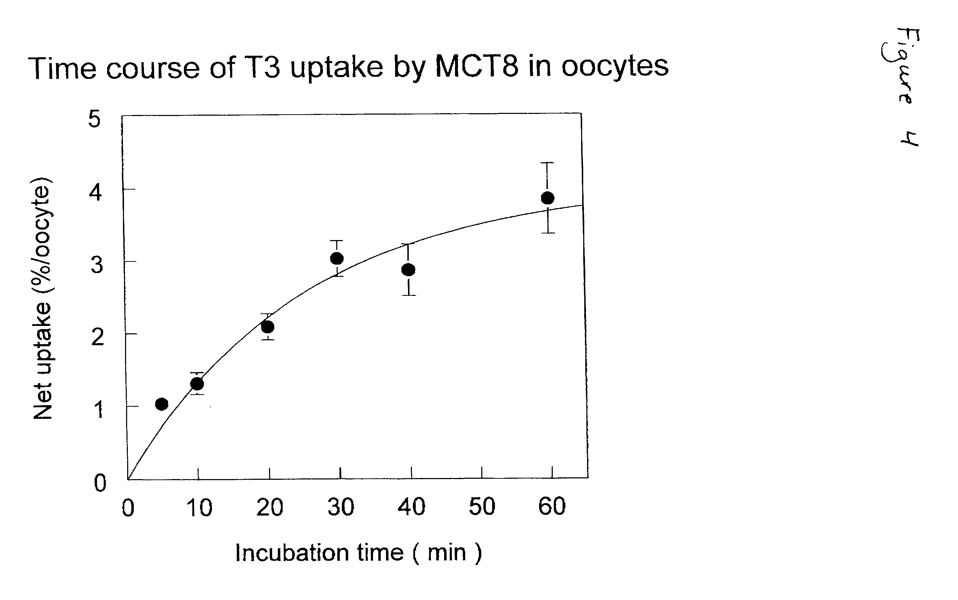

Xenopus oocytes were isolated and injected with rat MCT8 cRNA. After 3 days, groups of 10 injected or uninjected oocytes were incubated for 5-60 min at 25 C with 10 nM radioactive T3 in 0.1 ml medium. T3 uptake was determined as previously described (18). The results are shown in figure 4.

### Brief description of the drawings

Figure 1: Transport of iodothyronines by MCT8 in oocytes
Figure 2: Transport of amino acids by MCT8 in oocytes
Figure 3: Saturation of T4 transport by MCT8 in oocytes
Figure 4: Time course of T3 uptake by MCT8 in oocytes

### References

1. Larsen, P.R. and M.J. Berry. 1995. Nutritional and hormonal regulation of thyroid hormone deiodinases. *Annu. Rev. Nutr*. 15: 323-352.
2. Leonard, J.L. and J. Köhrle. 1996. Intracellular pathways of iodothyronine metabolism. *In* The Thyroid, L.E. Braverman and R. Utiger, editors. Lippincott-Raven, Philadelphia, 125-161.
3. St.Germain, D.L. and V.A. Galton. 1997. The deiodinase family of selenoproteins. *Thyroid* 7: 655-688.
4. Hennemann, G. and T.J. Visser. 1997. Thyroid hormone synthesis, plasma membrane transport and metabolism. *In* Handbook of Experimental Pharmacology, Vol 128; Pharmacotherapeutics of the Thyroid Gland, A.P. Weetman and A. Grossman, editors. Springer, Berlin, 75-117.
5. Oppenheimer, J.H., H.L. Schwartz and K.A. Strait. 1996. The molecular basis of thyroid hormone actions. *In* The Thyroid, L.E. Braverman and R. Utiger, editors. Lippincott-Raven, Philadelphia, 162-184.
6. Hennemann, G., M.E. Everts, M. de Jong, C.F. Lim, E.P. Krenning and R. Docter. 1998. The significance of plasma membrane transport in the bioavailablity of thyroid hormone. *Clin. Endocrinol*. 48: 1-8.
7. Hagenbuch, B., B. Stieger, M. Foguet, H. Lübbert and P.J. Meier. 1991. Functional expression cloning and characterization of the hepatocyte Na⁺/bile acid cotransport system. *Proc. Natl. Acad. Sci. USA*. 88: 10629-10633.
8. Jacquemin, E., B. Hagenbuch, B. Stieger, A.W. Wolkoff and P.J. Meier. 1994. Expression cloning of a rat liver Na⁺-independent organic anion transporter. *Proc. Natl. Acad. Sci. USA* 91: 133-137.
9. Hagenbuch, B. 1997. Molecular properties of hepatic uptake systems for bile acids and organic anions. J. *Membrane Biol*. 160: 1-8.
10. Meier, P.J. 1995. Molecular mechanisms of hepatic bile salt transport from sinusoidal blood into bile. Am. *J. Physiol*. 269: G801-G812.
11. Schroeder, A., U. Eckhardt, B. Stieger, R. Tynes, C.D. Schteingart, A.F. Hofmann, P.J. Meier and B. Hagenbuch. 1998. Substrate specificity of rat liver Na⁺-bile salt cotransporter in enopus laevis oocytes and CHO cells. *Am. J. Physiol*. 274: G370-G375.
12. Bossuyt, X., M. Müller and P.J. Meier. 1996. Multispecific amphipathic substrate transport by an organic anion transporter of human liver. J. *Hepatol.* 25: 733-738.
13. Bossuyt, X., M. Müller, B. Hagenbuch and P.J. Meier. 1996. Polyspecific drug and steroid clearance by an organic anion transporter of mammalian liver. *J. Pharmacol. Exp. Ther*. 276: 891-896.
14. Kanai, N., R. Lu, Y. Bao, A.W. Wolkoff and V.L. Schuster. 1996. Transient expression of oatp anion transporter in mammalian cells: identification of candidate substrates. *Am. J. Physiol*. 270: F319-F325.
15. Kullak-Ublick, G.A., T. Fisch, M. Oswald, B. Hagenbuch, P.J. Meier, U. Beuers and G. Paumgartner. 1998. Dehydroepiandrosterone sulfate (DHEAS): identification of a carrier protein in human liver and brain. *FEBS Lett*. 424: 173-176.
16. Henneman, G; Docter, R; Friesema, E; De Jong, M; Krenning, E; and Visser, Th. 2001. Plasma membrane transport of thyroid hormones and its role in thyroid hormone metabolism and bioavailability. Endocrine Reviews 22(4): 451-476.
17. Halestrap, A.P; and Price, N.T. 1999. The proton-linked monocarboxylate transporter (MCT) family: structure, function and regulation. Biochem.J. 343, 281-299.
18. Friesema, E; Docter, R; Moerings, E; Verrey, F; Krenning, E; Henneman, G; and Visser, Th. 2001. Thyroid hormone transport by the heterodimeric human system L amino acid transporter. Endocrinology 142(10): 4339-4348

## Claims

1. Use of a monocarboxylate transporter protein or a functional part, derivative and/or analogue thereof for altering transport of a thyroid hormone or a functional part, derivative and/or analogue thereof across a membrane.

2. An isolated molecule capable of specifically binding a monocarboxylate transporter protein or a functional part, derivative and/or analogue thereof, or a ligand of said monocarboxylate transporter protein.

3. Use of a molecule according to claim 2 for altering transport of a thyroid hormone or a functional part, derivative and/or analogue thereof across a membrane.

4. Use according to claim 1 or 3, wherein said monocarboxylate transporter protein comprises monocarboxylate transporter protein-8.

5. Use according to claim 1, 3 or 4 wherein said membrane comprises a plasma membrane.

6. A compound capable of influencing the binding or transporting of a ligand of, or capable of binding to, a plasma membrane polypeptide capable of transporting a thyroid hormone, wherein said polypeptide comprises a monocarboxylate transporter protein or a functional part, derivative and/or analogue thereof, for use as a medicament.

7. A compound according to claim 6 wherein said polypeptide is capable of transporting 3,3',5-triiodothyronine.

8. A compound according to claim 6 or 7 wherein said monocarboxylate transporter protein comprises monocarboxylate transporter protein-8.

9. Use of a compound according to anyone of claims 6-8 for the production of a medicament for the treatment of a thyroid hormone related disorder, non-thyroidal illness, obesity or cardiovascular illness.

10. A pharmaceutical composition comprising a compound according to anyone of claims 6-8, and a suitable carrier.

11. A method for altering transport of a thyroid hormone or a functional part, derivative and/or analogue thereof across a membrane comprising providing said membrane with a monocarboxylate transporter protein or a functional part, derivative and/or analogue thereof, and/or with a molecule capable of specifically binding a monocarboxylate transporter protein or a functional part, derivative and/or analogue thereof, or a ligand of said monocarboxylate transporter protein.

12. A method for treating a disorder such as a disorder of thyroid metabolism, non-thyroidal illness, obesity or cardiovascular illness, comprising administering a compound according to anyone of claims 6-8 or a pharmaceutical composition according to claim 10 to an individual suffering from said disorder.

13. Use of at least a functional part of a monocarboxylate transporter protein, or a nucleic acid encoding at least a functional part of a monocarboxylate transporter protein, in a method for treating a disorder such as a thyroid hormone related disorder such as a disorder of thyroid metabolism, non-thyroidal illness, obesity or cardiovascular illness.

14. Method according to claim 11 or 12 or use according to claim 13, comprising gene therapy.

15. Use of at least a functional part of a monocarboxylate transporter protein, or a nucleic acid encoding at least a functional part of a monocarboxylate transporter protein, in a method for detecting and/or generating a pharmaceutical compound.

16. A method according to claim 11,12 or 14 or a use according to claim 13-15 wherein said monocarboxylate transporter protein comprises monocarboxylate transporter protein-8.

17. A bioassay to identify or detect a candidate drug capable of binding to or influencing a plasma membrane polypeptide capable of transporting a thyroid hormone, wherein said polypeptide comprises a monocarboxylate transporter protein or a functional part, derivative and/or analogue thereof.

18. A bioassay according to claim 17 using at least a functional part of a monocarboxylate transporter protein, a nucleic acid encoding at least a functional part of a monocarboxylate transporter protein, a molecule capable of specifically binding at least part of said monocarboxylate transporter protein or a ligand thereof, a cell comprising said nucleic acid and/or a cell comprising at least part of said protein.

19. A bioassay according to claim 17 or 18 wherein said monocarboxylate transporter protein comprises monocarboxylate transporter protein-8.

20. An isolated monocarboxylate transporter-8 protein or a functional part, derivative and/or analogue thereof.

21. A vector comprising a nucleic acid sequence encoding a monocarboxylate transporter-8 protein or a functional part, derivative and/or analogue thereof, and/or a molecule capable of specifically binding a monocarboxylate transporter-8 protein or a functional part, derivative and/or analogue thereof, or capable of specifically binding a ligand of said monocarboxylate transporter-8 protein.

22. A gene delivery vehicle comprising a vector according to claim 21.
